# EUROPÄISCHE PATENTSCHRIFT

(11) **EP 2 985 339 B1**
(45) Veröffentlichungstag und Bekanntmachung des Hinweises auf die Patenterteilung: **13.05.2020**
(21) Anmeldenummer: 14002791.3
(22) Anmeldetag: 11.08.2014
(51) Int. Cl.: C12M 1/00, F26B 23/00

(54) **Gärrest-Konditionierer und Verfahren zur Konditionierung von Gärresten**
Fermentation residue conditioning device and method for conditioning fermentation residues
Conditionneur de résidus de fermentation et procédé de conditionnement de résidus de fermentation

(43) Veröffentlichungstag der Anmeldung: 17.02.2016
(73) Patentinhaber: Kompoferm GmbH, 33428 Marienfeld (DE)
(72) Erfinder: Eggersmann, Karlgünter, 33428 Marienfeld (DE)
(74) Vertreter: Schober, Mirko

(56) Entgegenhaltungen:
- EP-A1- 2 275 763
- EP-A2- 0 458 738
- WO-A1-03/042129
- WO-A1-2014/040662
- WO-A2-2008/095685
- DE-A1- 2 332 363
- DE-A1- 2 942 325
- DE-A1- 3 531 748
- DE-A1-102007 038 105
- DE-C- 492 226

## Beschreibung

Die Erfindung betrifft einen Gärrest-Konditionierer sowie ein Verfahren zur Konditionierung von Gärresten oder Klärschlamm und/oder organischen Restmassen mit hohem Wassergehalt, insbesondere aus der Fermentation von Hausmüll, Bioabfall und/oder Speisereste haltigen Ausgangsstoffen. Gärreste und Klärschlamm fallen beispielsweise in Anlagen zur Biogaserzeugung oder Kläranlagen an und weisen eine für ihre weitere Behandlung, beispielsweise durch Kompostierung, ungünstige Konsistenz, unter anderem einen zu hohen Feuchtigkeitsgehalt, auf. Dies gilt in besonderem Maße für Hausmüll, Bioabfall und/oder Speisereste haltige Ausgangsstoffe. Diese weisen zwar einen sehr hohen Energiegehalt auf, was sie für die Verwertung, beispielsweise mit Fermentationsprozessen mit anaerober Vergärung und anschließender Nachbehandlung, beispielsweise anschließender Kompostierung, geeignet erscheinen lässt. Nachteilig ist jedoch die Konsistenz solcher Ausgangsmaterialien sowie deren hoher Feuchtegehalt. Zur weiteren Behandlung wird solchen Ausgangsmaterialien häufige Luftporen bildendes und Feuchtigkeit aufnehmendes Strukturmaterial, z.B. der Siebüberlauf einer Kompostierung, insbesondere einer Bioabfallkompostierung und/oder zerkleinerter Grünabfall zugegeben. Die Mischung dieser Stoffe ist sehr häufig heterogen, was eine gleichmäßige Belüftung verhindert.

Auch weisen solche Ausgangsstoffe, insb. Hausmüll, Bioabfall und/oder Siebreste als Strukturmaterial aufweisende Ausgangsstoffe Störstoffe wie beispielsweise Plastikfolien, die aus Müllsäcken stammen, und/oder Netze auf, die möglichst nicht in die Fertigmaterialien, also beispielsweise den Kompost oder das Deponat gelangen sollen.

Aus diesen Gründen werden solche Ausgangsstoffe für weitere Behandlungsschritte konditioniert, d.h. insbesondere, es werden der Feuchtegehalt gesenkt, Ammoniak und/oder Methan ausgetrieben, Verklumpungen aufgelöst und/oder die Ausgangsstoffe homogenisiert.

Bei Trocknungsanlagen, wie es DE 10 2007 038 105 A1, WO 2008/095685 A2 und DE 35 31 748 A1 offenbaren, wird warme Luft durch das Trocknungsgut geleitet, denn warme Luft kann mehr Feuchtigkeit aufnehmen als kalte. Da hier nicht unmittelbar eine aerobe Behandlung des Materials unterstützt wird, muss die Temperatur nicht feingeregelt werden, es genügt, die Lufttemperatur in gewissen Grenzen zu regeln.

Bei der Behandlung von Gärresten, Klärschlamm und/oder organischen Restmassen, muss sowohl auf die Temperatur geachtet werden als auch auf die Feuchtigkeit. EP 2 275 763 A1 offenbart dazu zwei Belüftungssysteme, wobei mit Frischluft gemischte Abluft zugeführt und daneben ein Umluftsystem betrieben wird. Beide Luftsysteme können über Heizungen entsprechend temperiert werden, was die Belüftungsanlage komplex macht. Da es sich bei dem Verfahren um ein Batchsystem handelt, wird nichts über den Materialtransport mitgeteilt. WO 2014/040662 A1, WO2003/042 129 A1 und DE 49 22 26 A offenbaren aerobe Behandlungsvorrichtungen. Hier erfolgt die Belüftung zum einen über einen Umsetzer, der das Material beim Umsetzen kräftig durchmischt und dabei durchlüftet, als auch über eine Luftzufuhr von unten. Dabei kann der Luftdurchsatz über einen Temperatursensor zur Stabilisierung der Temperatur eingesetzt werden (DE 49 22 26 A).

Der Erfindung liegt daher die Aufgabe zugrunde, einen Gärrest-Konditionierer zur Aufbereitung derartiger Ausgangsstoffe sowie ein Verfahren zu deren Konditionierung aufzuzeigen, welche eine effiziente Konditionierung der Gärreste unter verminderter Freisetzung von Emissionen ermöglichen.

Die Aufgabe wird gelöst durch einen Gärrest-Konditionierer und ein Verfahren zur Konditionierung von Gärresten oder Klärschlamm nach den unabhängigen Ansprüchen. Die abhängigen Ansprüche betreffen vorteilhafte Ausführungsformen.

Im Folgenden wird für die Begriffe "Gärreste" und "Klärschlamm" sowie für "organische Restmassen mit hohem Wassergehalt, insbesondere Gärreste aus der Fermentation von Hausmüll, Siebresten und/oder Speisereste haltigen Ausgangsstoffen", lediglich der Begriff "Gärreste" verwendet. Dies gilt auch für die Ansprüche und dient der sprachlichen Vereinfachung.

Der erfindungsgemäße Gärrest-Konditionierer umfasst eine, sich vorzugsweise in einer ersten Erstreckungsrichtung erstreckende, Ablagefläche für Gärreste. Auf dieser können die Gärreste zu einem Haufwerk aufgeschichtet werden. Weiterhin umfasst er eine Gärrest-Aufgabestelle und eine, vorzugsweise von der Gärrest-Aufgabestelle entlang der ersten Erstreckungsrichtung beabstandete, Gärrest-Entnahmestelle. Gärrest-Aufgabestelle und Gärrest-Entnahmestelle bezeichnen dabei zunächst nur die räumlich festgelegten Bereiche, in denen die Gärreste auf den Gärrest-Konditionierer aufgegeben bzw. von diesem entnommen werden. Eine spezielle Ausgestaltung dieser Stellen kann vorteilhaft sein, ist jedoch grundsätzlich nicht notwendig. Gärrest-Aufgabestelle und Gärrest-Entnahmestelle müssen sich folglich konstruktiv nicht zwangsläufig vom Rest des Gärrest-Konditionierers, insbesondere anderen Abschnitten des Gärrest-Konditionierers entlang der ersten Erstreckungsrichtung unterscheiden.

Erfindungsgemäß ist die Förderung durch eine Ablagefläche zur unterseitigen Abstützung des Haufwerks bewirkbar, d.h. dass die für die Förderung des Gärrests benötigten Kräfte in die Unterseite des von dem Gärrest gebildeten Haufwerks eingeleitet werden.

Das Haufwerk ist dabei im weitestens Sinne als Haufwerk zu verstehen, insbesondere umfasst der Begriff auch sehr nasse Materialien, wie beispielsweise Klärschlamm, d.h. Materialien von stichfester bis pastöser Konsistenz, sowie sehr heterogene Materialien, insb. mit Verklumpungen und/oder durch flächige Gebilde diskret getrennten Bereichen, beispielsweise durch Plastikfolien eingeschlossene oder voneinander getrennte Bereiche, die beispielsweise von Müllsäcken herrühren. Der sprachlichen Einfachheit halber werden im Folgenden die räumlichen Anordnungen der genannten Ausgangsmaterialien im erfindungsgemäßen Gärrest-Konditionierer bzw. bei der Durchführung des erfindungsgemäßen Verfahrens allgemein als Haufwerke bezeichnet, auch wenn sie eine Konsistenz aufweisen, die nicht typisch für Haufwerke im engeren Sinne ist. Dies gilt auch für die Verwendung des Begriffs Haufwerke in den Ansprüchen.

Die Krafteinleitung von der Unterseite in das Haufwerk ermöglicht es, dieses durch den Gärrest-Konditionierer zu fördern, ohne dass es dabei auf die innere Struktur dieses Haufwerks ankommt. Damit lassen sich die verschiedensten Materialien problemlos fördern. Weiterhin ist die Förderung unabhängig von einer etwaigen Umschichtung bzw. Homogenisierung, d.h. es ist möglich, für eine Umschichtung oder Homogenisierung des Haufwerks entsprechende Einrichtungen vorzusehen und diese derart auf das Haufwerk abzustimmen, dass der Umschichtvorgang die Gegebenheiten des zu verarbeitenden Haufwerks berücksichtigt. So kann z.B. vorgesehen sein, dass die Umschichtung im Hinblick auf die Schonung von Plastikfolien und ähnlichen Störstoffen erfolgt. Hierdurch kann verhindert werden, dass diese zerkleinert und bei späteren Trennoperationen der falschen Fraktion zugeordnet werden, oder nicht abgetrennt werden. Auch in dieser Hinsicht ist die Krafteinleitung über die Unterseite des Haufwerks besonders vorteilhaft, da sie nicht mit einer Penetration des Haufwerks selbst einhergeht und dadurch ebenfalls Material schonend im Hinblick auf die Zerkleinerung möglicher Störstoffe ist.

Bei dem erfinderischen Gegenstand weist weiterhin die Ablagefläche gemäß unabhängigem Anspruch 1 hin und her bewegbare Ablageelemente zur Abstützung des Haufwerks auf. Diese sind insbsondere so ausgebildet, dass sich immer eine geschlossene, vorzugsweise auch eine flüssigkeitsdichte, Ablagefläche für das Haufwerk bildet. Dabei ist insbesondere in der quer zur Förderrichtung und/oder quer zur Bewegungsrichtung der Ablageelemente verlaufenden Richtung eine Mehrzahl Ablageelemente vorgesehen. Werden diese nun in ihrer Bewegung geeignet angesteuert, so können die Ablageelemente durch ihr Hin- und Herbewegen einen Transport des auf den Ablageelementen gelagerten Haufwerks bewirken, beispielsweise indem die Ablageelemente einzeln entgegen der Förderrichtung bewegt werden und dann gemeinsam in Förderrichtung, so dass das Haufwerk, welches auf den Ablageelementen gelagert ist, im Wesentlichen lediglich der Bewegung in Förderrichtung folgt. Entsprechend ist es auch möglich, dass die Ablageelemente gruppenweise bewegt werden, solange der gewünschte Fördereffekt erzielt wird. Eine derartige Ausgestaltung der Ablagefläche wird regelmäßig als Schubboden oder "walking floor" bezeichnet.

Zusätzlich oder alternativ zu den hin und her bewegbaren Ablageelementen sieht die Ablagefläche gemäß unabhängigem Anspruch 1 ein relativ zur Bodenfläche bewegbares Förderelement vor. Bei diesem kann es sich beispielsweise um ein dünnes Kratz-eisen handeln. Es hat sich gezeigt, dass wenn ein derartiges Förderelement flach genug gestaltet wird, es ebenfalls den Eintrag der Bewegungskraft nur an der Unterseite des Haufwerks im Sinne der Erfindung ermöglicht. Hierbei hat sich gezeigt, dass der gewünschte Effekt, d.h. Eintrag der die Bewegung bewirkenden Kraft ohne spürbar nachteilige Penetration des Haufwerks, mit derartigen beweglichen Förderelementen erreicht werden kann, wenn deren die Förderung unmittelbar bewirkender Teil nicht mehr als 40 mm, vorzugsweise 15 mm von der Ablagefläche in das Haufwerk hinein ragt bzw. sich in das Haufwerk hinein erstreckt. Unter dem die Förderung nicht unmittelbar bewirkenden Teil ist jeweils der Teil eines solchen beweglichen Förderelements zu verstehen, der in einem jeweiligen Zeitpunkt unmittelbar Kraft auf die Unterseite des Haufwerks ausübt. Es versteht sich, dass beispielsweise im Rahmen der Rückführung eines umlaufenden beweglichen Förderelements dieses beispielsweise an den Enden des Gärrest-Konditionierers nach oben oder unten aus diesem heraus und über oder unter dem Gärrest-Konditionierer zurück geführt werden kann, um so einen Endlosumlauf nach Art eines Förderbands zu erzeugen. Es versteht sich, dass ein solcher, von der Ablagefläche weg geführter Teil des Förderelements nicht als ein die Förderung unmittelbar bewirkender Teil des beweglichen Förderelements anzusehen ist.

Zusätzlich oder alternativ dazu kann die Ablagefläche gemäß unabhängigem Anspruch 1 auch nach Art eines Bandförderers ausgestaltet sein. Dieser verfügt vorzugsweise über flächige, vorzugsweise in Förderrichtung nebeneinander angeordnete Ablageelemente, welche sich bewegende Ablageflächen für das Haufwerk bilden. Die Ablageelemente sind dabei vorzugsweise mit ihrer größten Erstreckungsrichtung in Förderrichtung orientiert angeordnet.

Vorzugsweise sind die Ablageelemente, welche die Ablagefläche bilden bzw. die Ablagefläche aus Metall gebildet, was sich vorteilhaft auf eine vorteilhafte unterseitige Beheizung des Haufwerks auswirkt, da Metall eine gute Leitfähigkeit für Wärme besitzt.

Weiterhin umfasst die Erfindung, dass die Ablagefläche eine Heizeinrichtung aufweist, welche einen direkten Wärmeübertrag an das Haufwerk durch Wärmeleitung ermöglicht. Durch die Heizeinrichtung ist es möglich, das Haufwerk zu beheizen, was dessen Trocknung beschleunigt.

Der erfindungsgemäße Gärrest-Konditionierer ist zur Optimierung des thermischen Wirkungsgrades infolge von Wärmeverlusten an die Atmosphäre und somit Rückkondensation bevorzugt wärmegedämmt ausgebildet.

Weiterhin weist der Gärrest-Konditionierer vorzugsweise eine Umschicht- und Auflockerungseinrichtung auf. Diese Umschicht- und Auflockerungseinrichtung ist geeignet und bestimmt, die bei bestimmungsgemäßer Verwendung auf der Ablagefläche befindlichen Gärreste umzuschichten, zu homogenisieren, Verklumpungen aufzubrechen, neue Oberflächen zu bilden, einen Aufschluss des Gärrestes zu bewirken und/oder die Förderung des Gärrestes entlang der ersten Erstreckungsrichtung zu unterstützen.

Das erfindungsgemäße Verfahren sieht vorzugsweise vor, dass das Haufwerk im Bereich der Gärrest-Aufgabestelle einem Gärrest-Konditionierer aufgegeben wird und im Bereich einer, von der Gärrest-Aufgabestelle entlang einer ersten Erstreckungsrichtung einer Ablagefläche für das Haufwerk des Gärrest-Konditionierers beabstandeten, Gärrest-Entnahmestelle dem Gärrest-Konditionierer entnommen wird, wobei das Haufwerk von der Gärrest-Aufgabestelle zur Gärrest-Entnahmestelle entlang der ersten Erstreckungsrichtung über die Ablagefläche gefördert wird. Dabei ist es vorteilhaft, wenn die Umschicht- und Auflockerungseinrichtung zeitgleich nur auf einen Teilabschnitt der Gesamterstreckung der Ablagefläche entlang der ersten Erstreckungsrichtung zwischen Gärrest-Aufgabestelle und Gärrest-Entnahmestelle auf die Gärreste einwirkt. Die Umschicht- und Auflockerungseinrichtung, die sich dabei nur über einen Teilabschnitt der genannten Gesamterstreckung erstreckt, kann auf diese Weise konstruktiv kleiner, und damit Material und Kosten sparender ausgeführt werden, als eine Umschicht- und Auflockerungseinrichtung, welche auf die gesamte Gärrestmenge gleichzeitig einwirken könnte, sich dazu also über die gesamte Ablagefläche erstrecken müsste.

Dieser vorteilhaften Maßnahme liegt die Erkenntnis zugrunde, dass es im Hinblick auf den Konditionierungsprozess ausreichend ist, wenn nur jeweils ein Teil der Gärreste zeitgleich umgeschichtet wird, da insbesondere die Trocknungsvorgänge eine gewisse Zeitdauer beanspruchen. Das mehrfache Umschichten, welches dazu dient, die Gärrestmasse zu homogenisieren und insbesondere Verklumpungen aufzubrechen, beansprucht nur einen Bruchteil der Zeit, die der gesamte Konditionierungsprozess andauert.

Dies wird insbesondere durch eine Umschicht- und Auflockerungseinrichtung ermöglicht, die einen rotierenden Umschichtkörper aufweist. Die Umschicht- und Auflockerungseinrichtung wirkt über diesen Umschichtkörper, der vorzugsweise um eine horizontale und/oder rechtwinklig zur ersten Erstreckungsrichtung verlaufende Achse rotiert, auf die Gärreste ein. Dabei kann der Umschichtkörper einerseits eine Umschichtung bewirken, andererseits auch den Transport durch den Gärrest-Konditionierer unterstützen, wobei es besonders vorteilhaft ist, wenn die Rotationsrichtung des Umschichtkörpers so gewählt ist, dass sich dessen oberer Scheitelpunkt in Richtung der Gärrest-Entnahmestelle bewegt. Weiter wird durch diese Umschichtung beim mehrfachen Umschichten ein Gärrestklumpen nach und nach aufgebrochen, derart, dass äußeres bereits angetrocknetes Material abfällt und darunter befindliches noch feuchtes Material weiter angetrocknet wird, wobei sich dieser Vorgang wiederholt. Zudem werden Klumpen geteilt, so dass sich größere Oberflächen zur Trocknung einstellen. Unter einem Scheitelpunkt ist auch eine sich insbesondere bei einem walzenförmig ausgestalteten Umschichtkörper entstehende Scheitellinie zu verstehen.

Um es zu ermöglichen, dass die Umschicht- und Auflockerungseinrichtung zwar nicht zeitgleich aber zumindest zeitversetzt auf alle Bereiche des Haufwerks auf der Ablagefläche einwirken kann, ist es vorteilhaft, wenn die Umschicht- und Auflockerungseinrichtung entlang der ersten Erstreckungsrichtung verfahrbar ist.

Dies kann beispielsweise durch sich entlang der ersten Erstreckungsrichtung erstreckende Führungen, beispielsweise durch ein Schienensystem erreicht werden. Dabei befinden sich die Führungen vorteilhafterweise oberhalb der bei bestimmungsgemäßer Verwendung des Gärrest-Konditionierers sich ergebenden Haufwerkshöhe und/oder außerhalb des Gärrest-Konditionierers. Hierdurch wird sichergestellt, dass die Funktion der Führungen für die Umschicht- und Auflockerungseinrichtung nicht durch Gärreste beeinträchtigt wird.

Es ist vorteilhaft, das Verfahren so durchzuführen, dass sich eine Haufwerkshöhe des Haufwerks von 10 bis 100 cm, insbesondere von 20 bis 40 cm ergibt. Bei dieser Haufwerkshöhe ergibt sich eine Trockungsgeschwindigkeit, bei der sich die Vorteile der vorliegenden Erfindung effizient nutzen lassen.

Es ist sinnvoll, die Umschicht- und Auflockerungseinrichtung am Gärrest-Konditionierer, vorzugsweise an den Führungen, durch eine Abstandsänderungseinrichtung aufzunehmen. Die Abstandsänderungseinrichtung dient zur Veränderung des Abstands der Umschicht- und Auflockerungseinrichtung zur Ablagefläche und kann beispielsweise durch ein Schwenkelement realisiert sein. Hierdurch kann die Umschicht- und Auflockerungseinrichtung harten Gegenständen, die sich ggf. unter den Gärresten befinden, nach oben ausweichen, wodurch Beschädigungen der Umschicht- und Auflockerungseinrichtung bzw. der Ablagefläche durch die harten Gegenstände wirkungsvoll verhindert werden, wenn diese zwischen die Umschicht- und Auflockerungseinrichtung und die Ablagefläche geraten, und die Rückfahrt ermöglicht wird.

Wie oben bereits erwähnt, ist es durch die Heizeinrichtung möglich, das Haufwerk zu beheizen, was dessen Trocknung beschleunigt. Die Ablagefläche weist eine Heizeinrichtung auf, welche einem direkten Wärmeübertrag an das Haufwerk durch Wärmeleitung ermöglicht, da die Auflageelemente vom Wärmemedium durchströmt werden. Dabei ist insbesondere das Zusammenwirken einer beheizten Ablagefläche mit einer Umschicht- und Auflockerungseinrichtung vorteilhaft, da auf diese Weise mit jeder Umschichtung eine neue Oberfläche der Gärreste in Kontakt mit der beheizten Ablagefläche kommt, so dass sich insgesamt eine homogene und beschleunigte Trocknung ergibt.

Die Ablagefläche weist eine Belüftungseinrichtung zur Belüftung des Haufwerks auf.

Durch das Durchlüften des Haufwerks wird ein schnellerer Feuchtigkeitsabtransport und auch Wärmeeintrag in das Haufwerk, insbesondere mit vorgewärmter Luft, gewährleistet. Hierbei ist es insbesondere vorteilhaft, dass durch die Homogenisierung Verklumpungen, sogenannte Mikrochargen, aufgebrochen werden, so dass die Durchlüftung auch die innerhalb dieser Verklumpungen gespeicherte Feuchtigkeit erfassen kann und die Verklumpungen nicht umströmt.

Die Belüftungseinrichtung und/oder die Heizeinrichtung ist vorzugsweise entlang der ersten Erstreckungsrichtung des Gärrest-Konditionierers segmentiert. Das heißt, dass einzelnen Streckenabschnitten entlang der ersten Erstreckungsrichtung des Gärrest-Konditionierers jeweils eine eigene Belüftungeinrichtung und/oder Heizeinrichtung zugeordnet ist. Es kann auch eine Heizeinrichtung und/oder eine Belüftungseinrichtung mit einer Mehrzahl redundanter Elemente, beispielsweise einer Mehrzahl Vorlauf- oder Rücklauf- bzw. Zuluft- oder Abluftleitungen oder Kreisläufen vorgesehen sein, wobei die redundanten Elemente jeweils einzelnen Streckenabschnitten entlang der Längserstreckung des Gärrest-Konditionierers zugeordnet sind.

Bevorzugt ist vorgesehen, eine Anlage aus einer Mehrzahl Gärrest-Konditionierern vorzusehen. Die Mehrzahl Gärrest-Konditionierer kann dabei parallel oder/und in Reihe geschaltet sein. Bevorzugt ist eine Variante, bei der aus Bauraumgründen eine Mehrzahl von Gärrest-Kondtionieren räumlich übereinander angeordnet sind.

Der beschriebene Gärrest-Konditionierer bietet nicht nur die Möglichkeit, Gärreste thermisch zu trocknen, sondern stellt insbesondere eine Vorstufe für einen optimierten Start einer nachfolgenden aeroben Behandlung der Gärreste dar.

Mittels des beschriebenen Gärrest-Konditionierers wird in den Gärresten vorhandenes Ammoniak, das für einen aeroben Prozess toxisch ist, mittels vorgeheizter Luft effektiv ausgetrieben und sicher gefasst. Vorzugsweise ist der Gärrest-Konditionierer zur sicheren Fassung der Abluft und/oder Reduktion von Emissionen derart gestaltet, dass gasförmige Emissionen verhindert und/oder zumindest weitgehend vermieden werden. Insbesondere ist der Gärrest-Konditionierer gekapselt ausgeführt. Die aufkonzentrierten Abluftströme können einer entsprechenden Abluftbehandlung, z.B. einem sauren Wäscher, zugeführt werden.

Die Behandlung der Gärreste mittels des beschriebenen Gärrest-Konditionierers setzt durch den Luftstrom in den Gärresten vorhandenes Methan frei. Insbesondere im Hinblick auf die Möglichkeit, Ammoniak und/oder Methan aus dem Gärrest auszutreiben, ist es vorteilhaft, wenn der Gärrest-Konditionierer so gestaltet ist, dass es möglich ist, die Luft zum Belüften des Gärrests zumindest teilweise im Kreislauf zu führen. Vorzugsweise ist eine Regelung des Verhältnisses der im Kreislauf geführten Luft zu der zu- und/oder abgeführten Luft möglich. Auf diese Weise lässt sich der Wärmeeintrag und/oder die Beladung der abgeführten Luft mit Ammoniak und/oder Methan erhöhen. Dies hat den Vorteil, dass bei einer nachgeschalteten Abluftbehandlung ein geringeres Luftvolumen behandelt werden muss. Besonders vorteilhaft ist es, wenn der Regelbereich des Verhältnisses die Grenzfälle der reinen Kreislaufführung und/oder der reinen Frischluftbelüftung abdeckt.

Ebenso wird durch die mechanischen Komponenten des beschriebenen Gärrest-Konditionierers (Auflockern, Aufschließen und/oder Homogenisieren) eine Bildung von Methan innerhalb von Gärrestklumpen o.ä. deutlich reduziert bzw. vermieden und die Freisetzung des klimarelevanten Methan an die Atmosphäre in nachgelagerten Prozessen deutlich reduziert.

Durch eine optimierte Systematik/Steuerung der Umschicht- und Auflockerungseinheit besteht die Möglichkeit, Volumen- bzw. Schütthöhenverluste der Gärreste in Folge von z.B. des Abbaus von organischer Masse und/oder erhöhter Feinkornbildung durch die Bearbeitung auszugleichen. Über den Ausgleich der Schütthöhe der Gärreste hinaus kann während der Behandlung die Schütthöhe und damit die Verweilzeit der Gärreste innerhalb des Gärrest-Konditionierers erhöht werden. Dieses führt zu einer Reduktion der Investions- und damit Behandlungskosten.

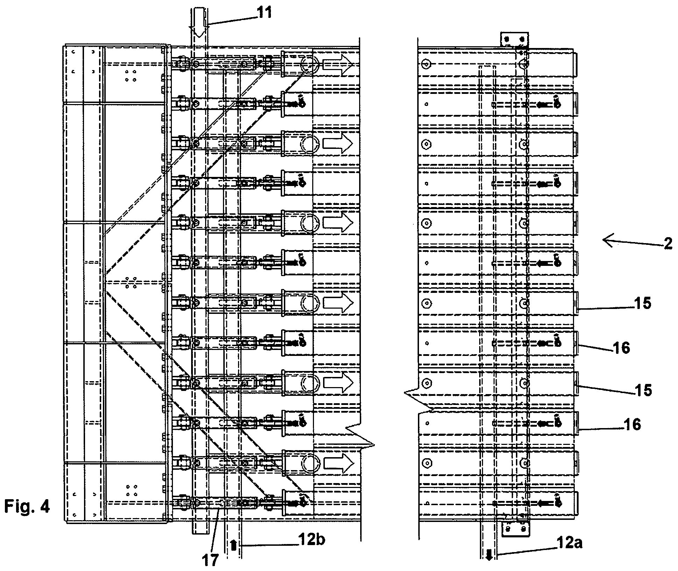

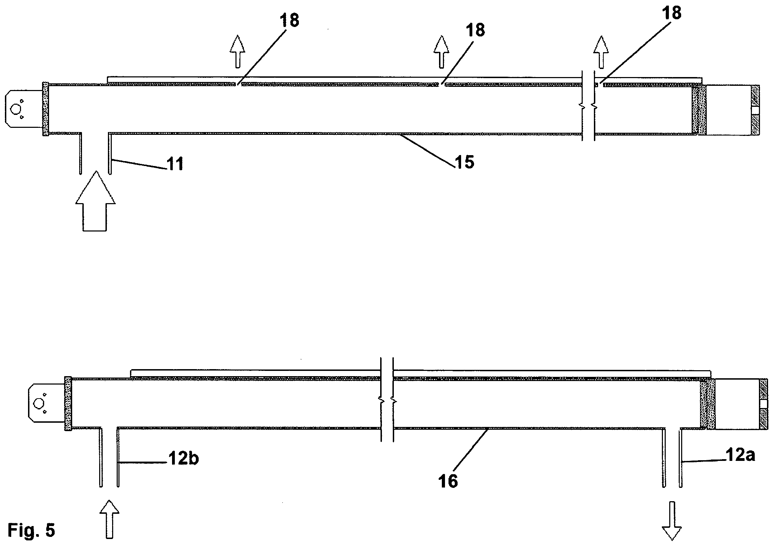

Die Erfindung wird im Folgenden anhand der Figuren 1 bis 5 schematisch näher erläutert.
- Figur 1: - zeigt einen beispielhaften erfindungsgemäßen Gärrest-Konditionierer in einer schematischen Darstellung,
- Figur 2: - zeigt einen Querschnitt eines beispielhaften erfindungsgemäßen Gärrest-Konditionierers mit rechtwinklig zur Förderrichtung verlaufender Schnittebene,
- Figur 3: - zeigt einen beispielhaften erfindungsgemäßen Gärrest-Konditionierer nach einem weiteren Ausführungsbeispiel, ebenfalls in Schnittdarstellung, mit der Schnittebene senkrecht zur Förderrichtung,
- Figur 4: - zeigt eine Detaildarstellung einer Ablagefläche eines beispielhaften erfindungsgemäßen Gärrest-Konditionierers,
- Figur 5: - zeigt Detaildarstellungen einzelner Ablageelemente eines beispielhaften erfindungsgemäßen Gärrest-Konditionierers.

Der beispielhafte erfindungsgemäße Gärrest-Konditionierer 1 weist eine Ablagefläche 2 für das Haufwerk 3 auf. Die Gärreste bilden das Haufwerk 3 auf der Ablagefläche 2 und werden an der Gärrest-Aufgabestelle 4, z.B. durch einen Förderer, auf die Ablagefläche 2 aufgegeben.

Die erfindungsgemäße Förderung des Haufwerks 3 zur Gärrest-Entnahmestelle 5 wird durch die Gestaltung der Ablagefläche 2 bewirkt. Diese weist im gezeigten Beispiel hin und her bewegbare Ablageelemente 15 bzw. 16 auf, denen im gezeigten Beispiel jeweils ein Antriebselement, beispielsweise eine Hydraulik 17, zum Hin- und Herbewegen der Ablageelemente 15 bzw. 16 zugeordnet ist.

Die Umschicht- und Auflockerungseinrichtung 6, die vorzugsweise entlang einem Schienensystem 8, welches oberhalb der Haufwerkshöhe der Gärreste und/oder außerhalb des Gärrest-Konditionierers angeordnet ist, aufgenommen ist, kann über einen in der Rotationsrichtung R rotierenden Umschichtkörper 7 auf die Gärreste einwirken, diese dabei umschichten, auflockern und aufbrechen.

Der Umschichtkörper 7 ist als um die Achse Y rotierende Walze ausgebildet, die Vorsprünge 7a aufweist, durch welche sie eine Profilierung erhält, mit der sie gut in die Gärreste 3 eingreifen und diese homogenisieren, aufbrechen und auflockern und somit schneller trocknen kann.

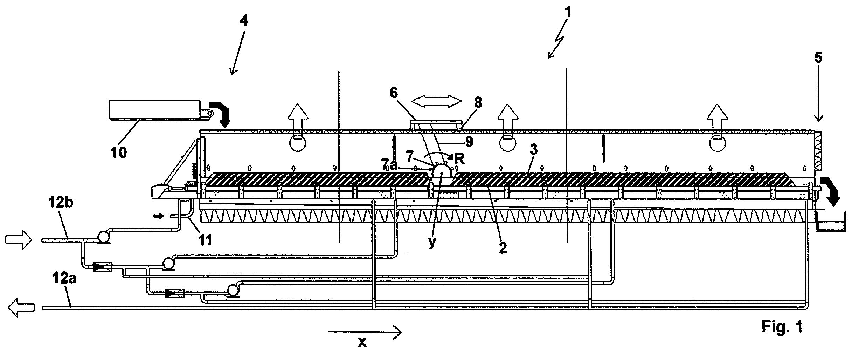

Der Boden des Gärrest-Konditionierers 1 weist unterhalb der Ablagefläche 2 eine Heizeinrichtung 12 und eine Belüftungseinrichtung 11 auf. Die Heizeinrichtung 12 weist von einem Heizmedium durchströmbare Kanäle auf, die unterhalb der Ablagefläche 2 horizontal verlaufen. Dabei sind die Kanäle jeweils mit einer Vorlaufleitung 12b und einer Rücklaufleitung 12a für das Heizmedium verbunden, die über eine gemeinsame Vorlaufleitung mit Heizmedium versorgt werden, wobei das Heizmedium ebenfalls über eine gemeinsame Rücklaufleitung wieder abgeführt werden kann. Natürlich können auch mehrere Heizkreisläufe mit entsprechenden Mehrzahlen von Vorlaufleitungen oder Rücklaufleitungen vorgesehen sein. Im gezeigten Beispiel in Figur 1 sind drei solcher Heizkreisläufe einzelnen Streckenabschnitten entlang der ersten Erstreckungsrichtung X des Gärrest-Konditionierers 1 zugeordnet.

Die Kanäle sind durch die Ablageelemente 16 gebildet, die vom Heizmedium durchströmt werden.

Der erfindungsgemäße Gärrest-Konditionierer weist ebenfalls eine Belüftungseinrichtung 11 auf. Im gezeigten Beispiel ist diese dadurch realisiert, dass Ablageelemente 15 mit Luftaustrittsöffnungen 18 versehen und durch die Belüftungseinrichtung 11 mit Zuluft versorgt werden, die aus den Luftaustrittsöffnungen 18 aus- und in das Haufwerk eintritt.

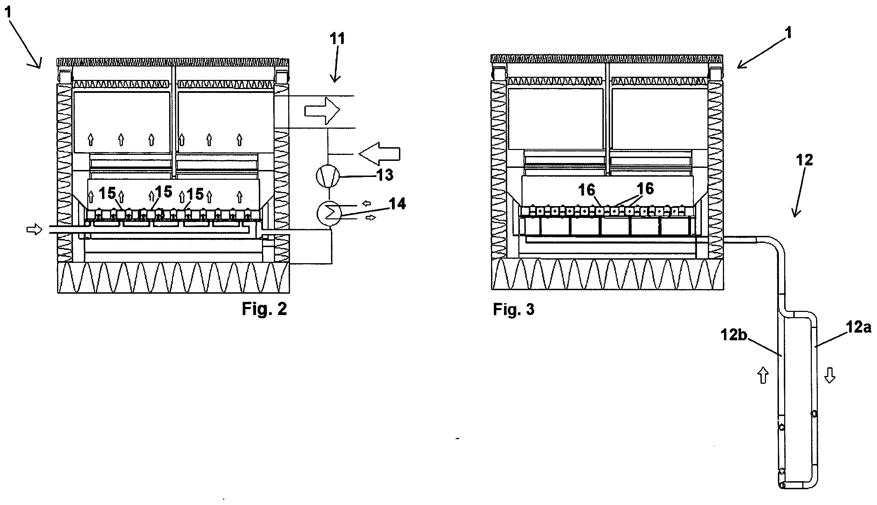

In Figur 2 ist eine entsprechende Belüftungseinrichtung 11 dargestellt. In Breitenrichtung des dort beispielhaft dargestellten Gärrest-Konditionierers wird jedes zweite Ablageelement 15 von der Belüftungseinrichtung 11 versorgt. Daneben ist in Figur 3 der Querschnitt eines beispielhaften erfindungsgemäßen Gärrest-Konditionierers abgebildet, bei dem jedes zweite Ablageelement 16 von einer Heizeinrichtung 12 mit einem Heizmedium versorgt wird. Ein einziger erfindungsgemäßer Gärrest-Konditionierer weist sowohl mit Luft durch eine Belüftungseinrichtung 11 versorgte Ablageelemente 15 als auch von einer Heizeinrichtung 12 mit einem Heizmedium versorgte und durchströmte Ablageelemente 16 auf. Diese können sich dann in Breitenrichtung des erfindungsgemäßen Gärrest-Konditionierers, also quer zur Förderrichtung X des Gärrest-Konditionierers, vorteilhafterweise einzeln und/oder in Gruppen abwechseln.

Im gezeigten Beispiel wird die Luft durch eine Fördereinrichtung 13, beispielsweise einen Verdichter, gefördert. Die Belüftungseinrichtung 11 ist im gezeigten Beispiel so ausgestaltet, dass ein Teil der Luft im Kreislauf geführt werden kann. Der Fördereinrichtung 13 kann ein Gemisch mit einem beliebigen Verhältnis aus Zuluft und Abluft, die dem Gärrest-Konditionierer 1 entnommen wird, zugeführt werden. Weiterhin ist eine zusätzliche Heizeinrichtung 14 für die zuzuführende Luft vorgesehen, was den Vorteil aufweist, dass so über die Luft zusätzliche Wärme in das Haufwerk der Gärreste 3 eingebracht werden kann. Weiterhin kann die warme Luft eine größere Menge Feuchtigkeit aufnehmen und aus den Gärresten abführen. Insbesondere bei Gärrest und Klärschlamm wird über die geschilderten Maßnahmen darin enthaltenes Ammoniak sicher ausgetrieben. Zudem werden durch das Aufbrechen der Gärrestklumpen dann noch eventuell ablaufende Methan produzierende Vergärungsprozesse bzw. anaerobe Prozesse sicher abgebrochen.

## Patentansprüche

1. Gärrest-Konditionierer (1) zur Konditionierung von Haufwerken (3) aus Gärresten, Klärschlamm und/oder organischen Restmassen mit hohem Wassergehalt, insbesondere von Gärresten aus der Fermentation von Hausmüll, Bioabfall und/oder Speisereste haltigen Ausgangsstoffen, mit einer Gärrest-Aufgabestelle (4) zur Einbringung des Haufwerks (3) und einer Gärrest-Entnahmestelle (5) zur Ausbringung des Haufwerks (3), wobei der Gärrest-Konditionierer (1) derart gestaltet ist, dass das Haufwerk (3) während der Konditionierung durch den Gärrest-Konditionierer (1) förderbar ist, wobei der Gärrest-Konditionierer (1) eine Belüftungseinrichtung (11), eine Heizeinrichtung (12) und eine Ablagefläche (2) zur unterseitigen Abstützung des Haufwerks (3) aufweist,
**dadurch gekennzeichnet,**
**dass** die Ablagefläche (2) derart gestaltet ist, dass durch sie die Förderung des Haufwerks (3) bewirkbar ist, indem die Ablagefläche (2) in Förderrichtung hin und her bewegbare Ablageelemente (15, 16) zur Abstützung des Haufwerks (3) aufweist und/oder indem ein relativ zur Ablagefläche (2) bewegliches Förderelement vorgesehen ist, welches zur Förderung des Haufwerks relativ zur Bodenfläche bewegbar und bevorzugt derart gestaltet ist, dass zumindest die die Förderung unmittelbar bewirkenden Teile sich nicht mehr als 40 mm von der Ablagefläche (2) in das Haufwerk (3) hinein erstrecken, und/oder indem die Ablagefläche (2) nach Art eines Bandförderers, insbesondere mit umlaufenden flächigen Ablageelementen, ausgebildet ist, wobei die Ablageelemente vorzugsweise in ihrer größten Erstreckungsrichtung in Förderrichtung (X) orientiert sind, und dass die Heizeinrichtung (12) so ausgelegt ist, dass sie einen direkten Wärmeübertrag an das Haufwerk (3) durch Wärmeleitung ermöglicht.

2. Gärrest-Konditionierer (1) nach Anspruch 1,
**dadurch gekennzeichnet,**
**dass** die Ablagefläche (2) als Schubboden ausgebildet ist.

3. Gärrest-Konditionierer (1) nach Anspruch 1 oder 2,
**dadurch gekennzeichnet,**
**dass** das bewegliche Förderelement ein Kratzeisen aufweist.

4. Gärrest-Konditionierer (1) nach einem der vorigen Ansprüche,
**dadurch gekennzeichnet,**
**dass** ein relativ zur Ablagefläche (2) bewegliches Förderelement vorgesehen ist, welches zur Förderung des Haufwerks relativ zur Bodenfläche bewegbar ist, wobei die die Förderung unmittelbar bewirkenden Teile des beweglichen Förderelements sich nicht mehr als 15 mm von der Ablagefläche (2) in das Haufwerk (3) hinein erstrecken.

5. Gärrest-Konditionierer (1) nach einem der vorigen Ansprüche,
**dadurch gekennzeichnet,**
**dass** der Gärrest-Konditionierer eine Umschicht- und Auflockerungseinrichtung (6) zur Umschichtung, Auflockerung, zum Aufschluss oder/und Homogenisieren eines auf der Ablagefläche (2) befindlichen Haufwerks aufweist.

6. Gärrest-Konditionierer (1) nach Anspruch 5,
**dadurch gekennzeichnet,**
**dass** die Umschicht- und Auflockerungseinrichtung (6) einen rotierenden, vorzugsweise um eine horizontale und/oder rechtwinklig zur ersten Erstreckungsrichtung (X) verlaufende Achse (Y) rotierenden, Umschichtkörper (7) aufweist.

7. Gärrest-Konditionierer (1) nach Anspruch 5 oder 6,
**dadurch gekennzeichnet,**
**dass** sich die Umschicht- und Auflockerungseinrichtung (6), insbesondere der Umschichtkörper (7), nur über einen Teilabschnitt der Gesamterstreckung der Ablagefläche entlang der ersten Erstreckungsrichtung (X) zwischen Gärrest-Aufgabestelle (4) und Gärrest-Entnahmestelle (5) erstreckt.

8. Gärrest-Konditionierer (1) nach einem der vorigen Ansprüche,
**dadurch gekennzeichnet,**
**dass** die Umschicht- und Auflockerungseinrichtung (6) entlang der ersten Erstreckungsrichtung (X) verfahrbar ist.

9. Gärrest-Konditionierer (1) nach einem der vorigen Ansprüche,
**dadurch gekennzeichnet,**
**dass** die Ablagefläche (2) die vorzugsweise flächige und/oder segmentierte Heizeinrichtung (12) und/oder eine Wärmedämmung aufweist.

10. Gärrestkonditionierer (1) nach einem der vorigen Ansprüche,
**dadurch gekennzeichnet,**
**dass** die Ablagefläche (2) die vorzugsweise flächige und/oder segmentierte Belüftungseinrichtung (11) zur Belüftung vorzugsweise mit Warmluft und/oder Teilumluftführung des Haufwerks aufweist.

11. Verfahren zur Konditionierung eines Gärrests, wobei der Gärrest als unterseitig abgestütztes Haufwerk (3) durch einen Gärrest-Konditionierer nach einem der vorigen Ansprüche, gefördert und der Gärrest während der Förderung für eine spätere, insbesondere aerobe Behandlung konditioniert wird,
**dadurch gekennzeichnet,**
**dass** die Förderung des Haufwerks (3) durch in die Unterseite des Haufwerks (3) eingeleitete Kräfte bewirkt wird.

12. Verfahren nach Anspruch 10 oder 11, **dadurch gekennzeichnet,**
**dass** das Haufwerk (3), insbesondere von der Unterseite des Haufwerks (3) aus, während der Förderung mit vorzugsweise vorgeheizter und/oder Kreislauf geführter Luft belüftet wird.

13. Verfahren nach einem der Ansprüche 10 bis 12, **dadurch gekennzeichnet,**
**dass** das Haufwerk (3) während der Förderung umgeschichtet, aufgelockert, aufgeschlossen und/oder homogenisiert wird.

## Claims

1. Fermentation residue conditioning device (1) for conditioning heaped materials (3) of fermentation residues, sewage sludge and/or organic residue substances having a high water content, in particular fermentation residues from the fermentation of source materials containing household refuse, biowaste and/or food residues, with a fermentation residue delivery point (4) for introducing the heaped material (3), and a fermentation residue removal point (5) for discharging the heaped material (3), wherein the fermentation residue conditioning device (1) is configured so that the heaped material (3) can be conveyed during conditioning through the fermentation residue conditioning device (1), wherein the fermentation conditioning device (1) has an air ventilation system (11), a heating system (12) and a shelf surface (2) for supporting the heaped material (3) from underneath,
**characterized in that**
the shelf surface (2) is configured so that the heaped material (3) can be conveyed thereby with the shelf surface (2) having shelf elements (15, 16) for supporting the heaped material (3), wherein these elements can be moved to and fro in the conveying direction, and/or wherein a conveying element is provided which is movable relative to the shelf surface (2) and which for conveying the heaped material can be moved relative to the base surface and is preferably configured so that at least the parts directly causing the conveyance extend no more than 40 mm from the shelf surface (2) into the heaped material (3), and/or wherein the shelf surface (2) is designed in the manner of a belt conveyor, in particular with revolving flat shelf elements, wherein the shelf elements are preferably oriented for most of their extension direction in the conveying direction (X), and that the heating system (12) is designed so that it enables a direct heat transfer to the heaped material (3) through heat conduction.

2. Fermentation residue conditioning device (1) according to claim 1,
**characterized in that**
the shelf surface (2) is designed as a walking floor.

3. Fermentation residue conditioning device (1) according to claim 1 or 2,
**characterized in that**
the movable conveying element has a scraper.

4. Fermentation residue conditioning device (1) according to one of the preceding claims
**characterized in that**
a conveying element is provided which is movable relative to the shelf surface (2) and which for conveying the heaped material is movable relative to the base surface, wherein the parts of the movable conveying element directly causing the conveyance extend no more than 15 mm from the shelf surface (2) into the heaped material (3).

5. Fermentation residue conditioning device (1) according to one of the preceding claims
**characterized in that**
the fermentation residue conditioning device has a redistributing and loosening system (6) for redistributing, loosening up, pulping and/or homogenizing a heaped material located on the shelf surface (2).

6. Fermentation residue conditioning device (1) according to claim 5,
**characterized in that**
the redistributing and loosening system (6) has a rotating redistributing body, which preferably rotates about a horizontal axis (Y) and/or an axis (Y) running at right angles to the first extension direction (X).

7. Fermentation residue conditioning device (1) according to claim 5 or 6
**characterized in that**
the redistributing and loosening system (6), in particular the redistributing body (7), extends only over a partial portion of the entire extension of the shelf surface along the first extension direction (X) between the fermentation residue delivery point (4) and the fermentation residue removal point (5).

8. Fermentation residue conditioning device (1) according to one of the preceding claims
**characterized in that**
the redistributing and loosening system (6) is movable along the first extension direction (X).

9. Fermentation residue conditioning device (1) according to one of the preceding claims **characterized in that**
the shelf surface (2) has the preferably flat and/or segmented heating device (12) and/or heat insulation.

10. Fermentation residue conditioning device (1) according to one of the preceding claims,
**characterized in that**
the shelf surface (2) has the preferably flat and/or segmented air ventilation system (11) for ventilation preferably with hot air and/or partial air circulation through the heaped material.

11. Method for conditioning a fermentation residue
wherein the fermentation residue is conveyed as a heaped material (3), supported underneath, through a fermentation residue conditioning device, according to one of the preceding claims, and the fermentation residue is conditioned during the conveyance for a subsequent, in particular aerobic, processing treatment,
**characterized in that**
the conveyance of the heaped material (3) is effected through forces introduced into the underneath of the heaped material (3).

12. Method according to claim 10 or 11,
**characterized in that**
the heaped material (3) is ventilated, in particular from underneath the heaped material (3), during the conveyance, with preferably preheated and/or circulating air.

13. Method according to one of claims 10 to 12,
**characterized in that**
during the conveyance the heaped material (3) is redistributed, loosened up, pulped and/or homogenized.

## Revendications

1. Conditionneur de résidu de fermentation (1) pour le conditionnement de tas (3) de résidu de fermentation, de boue d'épuration et / ou de masses de résidus organiques ayant une teneur en eau élevée, en particulier de résidu de fermentation résultant de la fermentation d'ordures ménagères, de résidus biologiques et / ou de matières contenant des restes d'aliments, avec un poste de dépose de résidu de fermentation (4) pour la prise en charge du tas (3) et un poste de prélèvement de résidu de fermentation (5) pour l'enlèvement du tas (3), sachant que le conditionneur de résidu de fermentation (1) est réalisé de manière à ce que le tas (3) puisse être convoyé dans le conditionneur de résidu de fermentation (1) pendant le conditionnement, sachant que le conditionneur de résidu de fermentation (1) présente une installation de ventilation (11), une installation de chauffage (12) et une surface de support (2) pour soutenir ie tas (3) par en bas,
**caractérisé en ce que**
la surface de support (2) est réalisée de manière à ce que le convoyage du tas (3) soit faisable pas elle-même, du fait que la surface de support (2) présente des éléments de support (15, 16), qui, pouvant être déplacés en va-et-vient dans la direction de convoyage, sont destinés à soutenir ie tas (3), et / ou du fait qu'est prévu un élément de convoyage qui peut être déplacé par rapport à la surface du fond pour le convoyage du tas et qui est de préférence réalisé de manière à ce que tout au moins les pièces assurant directement le convoyage ne s'étendent pas dans le tas (3) de plus de 40 mm à partir de la surface de support (2) et / ou du fait que la surface de support (2) est réalisée à la manière d'une bande transporteuse, en particulier dotée d'éléments de support plats, continus, sachant que les éléments de support sont orientés de préférence avec le sens de leur plus grande dimension dans la direction de convoyage (X) et que l'installation de chauffage (12) est conçue pour permettre la transmission directe de la chaleur au tas (3).

2. Conditionneur de résidu de fermentation (1) selon la revendication 1,
**caractérisé en ce que**
la surface de support (2) est réalisée en forme de fond coulissant.

3. Conditionneur de résidu de fermentation (1) selon la revendication 1 ou 2,
**caractérisé en ce que**,
l'élément de convoyage mobile présente un racloir.

4. Conditionneur de résidu de fermentation (1) selon l'une des revendications précédentes,
**caractérisé en ce que**,
pour le convoyage, est prévu un élément de convoyage déplaçable par rapport à la surface de support (2), lequel peut être déplacé par rapport à la surface de fond, sachant que les pièces des éléments de convoyage mobiles assurant directement le convoyage ne s'étendent pas dans ie tas (3) de plus de 15 mm à partir de la surface de support (2).

5. Conditionneur de résidu de fermentation (1) selon l'une des revendications précédentes,
**caractérisé en ce que**
le conditionneur de résidu de fermentation (1) présente une surface de retournement de tas et d'ameublissement (6) pour désagglomérer et / ou homogénéiser un tas se trouvant sur la surface de support (2) .

6. Conditionneur de résidu de fermentation (1) selon la revendication 5,
**caractérisé en ce que**
la surface de retournement de tas et d'ameublissement (6) présente un corps de retournement de tas (7) rotatif, qui tourne autour d'un axe (Y) de préférence horizontal et / ou s'étendant à angle droit par rapport à la première direction d'extension (X).

7. Conditionneur de résidu de fermentation (1) selon revendication 5 ou 6,
**caractérisé en ce que**
l'installation de retournement de tas et d'ameublissement (6), en particulier le corps de retournement de tas (7), s'étend seulement sur une section partielle de l'étendue totale de la surface de support le long d'une première direction d'extension (X), entre le poste de dépose de résidu de fermentation (4) et le poste de prélèvement de résidu de fermentation (5).

8. Conditionneur de résidu de fermentation (1) selon l'une des revendications précédentes,
**caractérisé en ce que**
l'installation de retournement du tas et d'ameublissement (6) peut être conduite le long de la première direction d'extension (X)

9. Conditionneur de résidu de fermentation (1) selon l'une des revendications précédentes,
**caractérisé en ce que**
la surface de support (2) est pourvue de l'installation de chauffage (12), de préférence plate et / ou segmentée, et / ou d'une isolation thermique.

10. Conditionneur de résidu de fermentation (1) selon l'une des revendications précédentes,
**caractérisé en ce que**
la surface de support (2) est pourvue de l'installation de ventilation (11), de préférence plate et / ou segmentée, pour la ventilation du tas, de préférence à l'air chaud, et / ou par circulation d'air partielle.

11. Procédé de conditionnement d'un résidu de fermentation, sachant que le résidu, en tant que tas (3) soutenu par en bas, est convoyé par un conditionneur de résidu de fermentation selon l'une des revendications précédentes et que le résidu de fermentation est conditionné pendant le convoyage pour un traitement ultérieur, de préférence aérobie,
**caractérisé en ce que**
le convoyage du tas (3) est effectué par des forces qui sont induites par la face inférieure du tas (3).

12. Dispositif selon revendication 10 ou 11,
**caractérisé en ce que**,
pendant le convoyage, le tas (3) est ventilé, en particulier par le côté inférieur du tas (3), de préférence avec de l'air préchauffé et / ou conduit en circuit fermé.

13. Dispositif selon l'une des revendications 10 à 12,
**caractérisé en ce que**
le tas (3), pendant le convoyage, est retourné, ameubli, désaggloméré et / ou homogénéisé.
